(19) Europäisches Patentamt
European Patent Office
Office européen des brevets

(11) **EP 3 029 077 B1**

(12) # EUROPEAN PATENT SPECIFICATION

(45) Date of publication and mention of the grant of the patent:
**19.08.2020 Bulletin 2020/34**

(21) Application number: **14831578.1**

(22) Date of filing: **17.07.2014**

(51) Int Cl.:
***C08F 2/32*** *(2006.01)*
***C08F 20/00*** *(2006.01)*
***C08K 5/10*** *(2006.01)*
***C08L 101/14*** *(2006.01)*
***A61L 15/48*** *(2006.01)*
***A61L 15/60*** *(2006.01)*
***C08F 2/20*** *(2006.01)*
***C08K 5/103*** *(2006.01)*
***C08L 71/02*** *(2006.01)*
***C08K 5/1535*** *(2006.01)*

(86) International application number:
**PCT/JP2014/069013**

(87) International publication number:
**WO 2015/016075 (05.02.2015 Gazette 2015/05)**

(54) **PROCESS FOR PRODUCING WATER-ABSORBENT RESIN PARTICLES**

VERFAHREN ZUR HERSTELLUNG EINES WASSERABSORBIERENDEN HARZPARTIKELS

PROCÉDÉ DE PRODUCTION DE PARTICULE DE RÉSINE ABSORBANT L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.07.2013 JP 2013156875**

(43) Date of publication of application:
**08.06.2016 Bulletin 2016/23**

(73) Proprietor: **Sumitomo Seika Chemicals Co., Ltd.**
**Kako-gun, Hyogo 675-0145 (JP)**

(72) Inventor: **TAKATORI, Junichi**
**Himeji-shi,**
**Hyogo 672-8076 (JP)**

(74) Representative: **Elkington and Fife LLP**
**Prospect House**
**8 Pembroke Road**
**Sevenoaks, Kent TN13 1XR (GB)**

(56) References cited:
**EP-A1- 2 011 803**
**EP-A2- 0 349 240**
**EP-A2- 0 349 241**
**WO-A1-2012/066888**
**JP-A- H06 345 819**
**JP-A- 2009 132 755**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

TECHNICAL FIELD

The present invention relates to a process for producing water-absorbent resin particles, in particular, to a process for producing water-absorbent resin particles through a reversed-phase suspension polymerization of a water-soluble ethylenically unsaturated monomer.

BACKGROUND ART

In various kinds of absorbent articles such as a disposable diaper, a sanitary napkin, an incontinence pad and a pet sheet, and various kinds of absorbent products such as a water-retaining material for soil, a water-blocking material for power cable and a dew-catcher, water-absorbent resin particles have been used. For example, absorbent articles such as a disposable diaper is generally formed by allowing an absorbent material including water-absorbent resin particles and hydrophilic fiber to be held between a liquid-permeable sheet arranged at a side with which a human body is brought into contact and a liquid-impermeable sheet arranged at the opposite side. The absorbent material used herein is produced, for example, by allowing a mixture including water-absorbent resin particles and crushed hydrophilic fiber to be layered on a metal mesh by means of an air flow, and then, allowing the layered product to be compressed by pressing.

As water-absorbent resin particles usable in absorbent articles or absorbent products, for example, a hydrolyzate of a starch-acrylonitrile graft copolymer, a neutralized product of a starch-acrylic acid graft copolymer, a saponified product of a vinyl acetate-acrylic acid ester copolymer, a crosslinked product of a partially neutralized polymer of acrylic acid, a partially neutralized product of polyacrylic acid, and the like have been known.

Water-absorbent resin particles to be used for the absorbent material are required to have a moderate particle size and a narrow particle size distribution in addition to being satisfactory in water-absorption ability such as water-retention capacity. In the case where there are many particles having a large particle size in the water-absorbent resin particles, the absorbent material is liable to become hard when being compressed. Contrarily, in the case where there are many particles having a small particle size in the water-absorbent resin particles, some of the particles are liable to pass through openings of the metal mesh in the process of producing the absorbent material and accordingly a loss of the particles occurs. Therefore, it is desirable that the water-absorbent resin particles used for the absorbent material have a median particle size suitable for a design for an intended absorbent material or absorbent article, and have a narrow particle size distribution.

As a production method of water-absorbent resin particles, from the viewpoints of the high level of performance of the resulting water-absorbent resin particles and the simplicity of the production method, methods of allowing a water-soluble ethylenically unsaturated monomer to undergo a polymerization are the mainstream. Examples of the polymerization method include an aqueous polymerization method of subjecting an aqueous solution of a water-soluble ethylenically unsaturated monomer to a polymerization and allowing the water-containing gelated product thereby obtained to be milled and dried, a reversed-phase suspension polymerization method of allowing a water-soluble ethylenically unsaturated monomer to be dispersed in the presence of a dispersion stabilizer in an organic solvent such as a hydrocarbon dispersion medium to undergo a suspension polymerization and allowing the water-containing gelated product thereby obtained to be dehydrated and dried, and the like.

In the aqueous polymerization method, the water-containing gel obtained by the polymerization is a viscous block-shaped material and thereby makes the milling process and drying process complicated. In addition, water-absorbent resin particles having a moderate particle size and a narrow particle size distribution are hardly obtained since fine particles are easily generated in the milling process.

In contrast, in the reversed-phase suspension polymerization method, it is possible to control the size of the water-absorbent resin particle by adjusting the size of the droplet of a water-soluble ethylenically unsaturated monomer dispersed in a hydrocarbon dispersion medium. Consequently, with regard to water-absorbent resin particles, there have been proposed various methods of particle size control by the reversed-phase suspension polymerization method.

For example, as the method for producing water-absorbent resin particles having a narrow particle size distribution, there have been proposed a method of using a sorbitan fatty acid ester as a dispersion stabilizer (see Patent Literature 1), a method of using a sorbitan fatty acid ester with an HLB of 8 to 12 as a dispersion stabilizer (see Patent Literature 2), a method of using a polyglycerol fatty acid ester as a dispersion stabilizer (see Patent Literature 3), and the like.

However, in these production methods, it is difficult to obtain water-absorbent resin particles achieving both satisfactory water-absorption ability and a preferred state of particles.

PRIOR ART LITERATURES

PATENT LITERATURES

Patent Literature 1: Japanese Patent Application Laid-Open 56-26909
Patent Literature 2: Japanese Patent Application Laid-Open 56-131608
Patent Literature 3: Japanese Patent Application Laid-Open 62-172006

SUMMARY OF THE INVENTION

PROBLEMS TO BE SOLVED BY THE INVENTION

The present invention intends to obtain water-absorbent resin particles achieving both satisfactory water-absorption ability and a preferred state of particles by polymerization of a water-soluble ethylenically unsaturated monomer.

MEANS FOR SOLVING PROBLEMS

The present inventors have found that, in the case of allowing a water-soluble ethylenically unsaturated monomer to undergo a polymerization by a reversed-phase suspension polymerization method to produce water-absorbent resin particles, water-absorbent resin particles achieving both satisfactory water-absorption ability and a preferred state of particles can be obtained when one which contains a tall oil fatty acid ester is used as a dispersion stabilizer, and thus, the present invention has been completed.

Accordingly, a process for producing water-absorbent resin particles according to the present invention includes the step of allowing a water-soluble ethylenically unsaturated monomer to undergo a reversed-phase suspension polymerization in the presence of a dispersion stabilizer, using a radical polymerization initiator in a hydrocarbon dispersion medium. The dispersion stabilizer usable herein contains a tall oil fatty acid ester. The tall oil fatty acid ester is usually at least one kind of ester selected from the group consisting of a sorbitol tall oil fatty acid ester, a sorbitan tall oil fatty acid ester and a polyethylene glycol tall oil fatty acid ester.

In this production process, it is preferred that the amount of the dispersion stabilizer used relative to 100 parts by mass of the water-soluble ethylenically unsaturated monomer be set to 0.1 to 30 parts by mass.

In one embodiment of this production process, the reversed-phase suspension polymerization is performed by a multi-stage process of two or more stages.

Moreover, this production process may further include the step of subjecting the water-absorbent resin particles obtained by the reversed-phase suspension polymerization to post-crosslinking.

Since a dispersion stabilizer containing a tall oil fatty acid ester is used in the production process according to the present invention, it is possible to produce water-absorbent resin particles achieving both satisfactory water-absorption ability and a preferred state of particles, which are attributed to a polymerized product of the water-soluble ethylenically unsaturated monomer.

Other objects or advantageous results of the present invention will be described in the detailed description below.

EMBODIMENTS OF THE INVENTION

The process for producing water-absorbent resin particles according to the present invention is to polymerize a water-soluble ethylenically unsaturated monomer.

Examples of the water-soluble ethylenically unsaturated monomer usable in the present invention include acrylic acid and a salt thereof, methacrylic acid and a salt thereof, 2-acrylamide-2-methylpropanesulfonic acid and a salt thereof, 2-methacrylamide-2-methylpropanesulfonic acid and a salt thereof, a nonionic monomer such as acrylamide, methacrylamide, N,N-dimethylacrylamide, N,N-dimethylmethacrylamide, 2-hydroxyethyl acrylate, 2-hydroxyethyl methacrylate, N-methylolacrylamide, N-methylolmethacrylamide, polyethylene glycol monoacrylate and polyethylene glycol monomethacrylate, an amino group-containing unsaturated monomer such as N,N-diethylaminoethyl acrylate, N,N-diethylaminoethyl methacrylate, N,N-diethylaminopropyl acrylate, N,N-diethylaminopropyl methacrylate, diethylaminopropyl acrylamide and diethylaminopropyl methacrylamide, a quaternized product of the amino group-containing unsaturated monomer, and the like. Two or more kinds of these water-soluble ethylenically unsaturated monomers may be used in combination.

Among the water-soluble ethylenically unsaturated monomers exemplified above, preferred are acrylic acid and a salt thereof, methacrylic acid and a salt thereof, acrylamide, methacrylamide, N,N-dimethylacrylamide and N,N-dimethylmethacrylamide since these are industrially easily available. In particular, since water-absorbent resin particles having high water-absorption ability are easily obtained, acrylic acid and a salt thereof and methacrylic acid and a salt thereof are preferred.

In the case where a water-soluble ethylenically unsaturated monomer is one having an acid group such as acrylic acid, methacrylic acid, 2-acrylamide-2-methylpropanesulfonic acid and 2-methacrylamide-2-methylpropanesulfonic acid, the water-soluble ethylenically unsaturated monomer may be one prepared by allowing the acid group to be previously neutralized with an alkaline neutralizing agent as necessary. Although the alkaline neutralizing agent used for the neutralization of acid groups is not particularly limited, examples thereof include an alkali metal salt such as sodium hydroxide, sodium carbonate, sodium hydrogen carbonate, potassium hydroxide and potassium carbonate, ammonia and the like. Two or more kinds of these alkaline neutralizing agents may be used in combination. As the alkaline neutralizing agent, one in the state of being an aqueous solution may be used for the purpose of making the neutralization operation of the water-soluble ethylenically unsaturated monomer simple.

The neutralization degree of the water-soluble ethylenically unsaturated monomer with the alkaline neutralizing agent is not particularly limited. However, from the viewpoints of heightening the osmotic pressure of the resulting water-absorbent resin particles to enhance the water-absorption ability and preventing problems on safety and the like attributed to the existence of an excessive alkaline neutralizing agent, it is preferred that the neutralization degree against all acid groups which the water-soluble ethylenically unsaturated monomer has is set to 10 to 100% by mole, in particular to 30 to 80% by mole.

To the water-soluble ethylenically unsaturated monomer to be polymerized, an internal-crosslinking agent can be previously added as necessary. Examples of the usable internal-crosslinking agent include an unsaturated polyester obtained by allowing a polyol such as a diol or a triol including ethylene glycol, polyethylene glycol, propylene glycol, polypropylene glycol, 1,4-butanediol, trimethylolpropane, glycerol, polyglycerol or the like and an unsaturated acid such as acrylic acid, methacrylic acid, maleic acid or fumaric acid to undergo a reaction; a bisacrylamide such as N,N'-methylene bisacrylamide; a diacrylate, a dimethacrylate, a triacrylate or a trimethacrylate obtained by allowing a polyepoxide and acrylic acid or methacrylic acid to undergo a reaction; a carbamyl diacrylate or a carbamyl dimethacrylate obtained by allowing a polyisocyanate such as tolylene diisocyanate or hexamethylene diisocyanate and hydroxyethyl acrylate or hydroxyethyl methacrylate to undergo a reaction; a compound having two or more polymerizable unsaturated groups such as allylated starch, allylated cellulose, diallyl phthalate, N,N',N''-triallyl isocyanate and divinylbenzene; a polyglycidyl compound such as a diglycidyl compound or a triglycidyl compound including ethylene glycol diglycidyl ether, polyethylene glycol diglycidyl ether, propylene glycol diglycidyl ether, polypropylene glycol diglycidyl ether, glycerol diglycidyl ether, polyglycerol diglycidyl ether, and the like; an epihalohydrin compound such as epichlorohydrin, epibromohydrin and $\alpha$-methylepichlorohydrin; a compound having two or more reactive functional groups such as an isocyanate compound including 2,4-tolylene diisocyanate, hexamethylene diisocyanate, and the like; an oxetane compound such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol and 3-butyl-3-oxetaneethanol, and the like. Two or more kinds of these internal-crosslinking agents may be used in combination.

For the purpose of sufficiently enhancing the water-absorption ability of the intended water-absorbent resin particles, it is preferred that the amount of the internal-crosslinking agent used is set to 0.00001 to 1 mol, in particular to 0.0001 to 0.5 mol, relative to 100 mol of the water-soluble ethylenically unsaturated monomer.

In the polymerization of the water-soluble ethylenically unsaturated monomer, a water-soluble ethylenically unsaturated monomer is dispersed in a hydrocarbon dispersion medium and the water-soluble ethylenically unsaturated monomer is allowed to undergo a reversed-phase suspension polymerization using a radical polymerization initiator in the presence of a dispersion stabilizer.

In the reversed-phase suspension polymerization, the water-soluble ethylenically unsaturated monomer may be added directly to a hydrocarbon dispersion medium. For the purpose of enhancing the dispersion efficiency in the hydrocarbon dispersion medium, the water-soluble ethylenically unsaturated monomer may be added to the hydrocarbon dispersion medium as an aqueous solution. In the case where an aqueous solution of the water-soluble ethylenically unsaturated monomer is added to a hydrocarbon dispersion medium, a concentration of the water-soluble ethylenically unsaturated monomer in the aqueous solution is not particularly limited. Usually, it is preferred that the concentration is set to a concentration greater than or equal to 20% by mass and less than or equal to a saturated concentration. In particular, it is preferred that the concentration thereof be set to 25 to 70% by mass, and it is more preferred that the concentration thereof be set to 30 to 55% by mass.

In cases of using an aqueous solution of the water-soluble ethylenically unsaturated monomer, when the revolution number of stirring at the time of the polymerization is common to the cases, there is a tendency that the more the viscosity of the aqueous solution of the water-soluble ethylenically unsaturated monomer is increased, the larger the particle size of the resulting water-absorbent resin particles becomes. On that account, the aqueous solution of the water-soluble ethylenically unsaturated monomer may contain a thickener for the purpose of adjusting the particle size of the target water-absorbent resin particles. Examples of the thickener used for such a purpose include hydroxyethyl cellulose, hydroxypropyl cellulose, methyl cellulose, carboxymethyl cellulose, polyacrylic acid, a neutralized product of polyacrylic acid, a partially neutralized product of polyacrylic acid, polyethylene glycol, polyacrylamide, polyethyleneimine, dextrin, sodium alginate, polyvinyl alcohol, polyvinyl pyrrolidone, polyethylene oxide, and the like.

In the reversed-phase suspension polymerization, it is also possible to allow a water-soluble ethylenically unsaturated monomer to undergo a polymerization by multi-stages having two or more stages in a hydrocarbon dispersion medium. In this case, water-absorbent resin particles already formed in the polymerization reaction system can be agglomerated by a polymerization of a water-soluble ethylenically unsaturated monomer added at a subsequent stage, whereby water-absorbent resin particles having a large particle size can be produced. According to such a multi-stage polymerization process, for example, water-absorbent resin particles having a relatively large particle size, which are suitable for absorbent articles such as a disposable diaper, can be easily produced.

In the case where the reversed-phase suspension polymerization is performed by multi-stages, a water-soluble ethylenically unsaturated monomer added at each stage may be the same as or different from one added at another stage. Moreover, as the water-soluble ethylenically unsaturated monomer added at each stage, one which contains an internal-crosslinking agent and one which does not contain an internal-crosslinking agent can be appropriately selected.

A hydrocarbon dispersion medium usable in the production process of the present invention is not particularly limited as long as it is one usable in the reversed-phase suspension polymerization of a water-soluble ethylenically unsaturated monomer. Examples of the usable hydrocarbon dispersion medium include an aliphatic hydrocarbon with 6 to 8 carbon atoms such as n-hexane, n-heptane, 2-methylhexane, 3-methylhexane, 2,3-dimethylpentane, 3-ethylpentane and n-octane; an alicyclic hydrocarbon such as cyclohexane, methylcyclohexane, cyclopentane, methylcyclopentane, trans-1,2-dimethylcyclopentane, cis-1,3-dimethylcyclopentane and trans-1,3-dimethylcyclopentane; an aromatic hydrocarbon such as benzene, toluene and xylene, and the like. Two or more kinds of these hydrocarbon dispersion media may be used in combination.

Among the hydrocarbon dispersion media, preferred is n-hexane, n-heptane or cyclohexane since these are industrially easily available, the quality thereof is stable, and furthermore, these are inexpensive. In the case where two or more kinds of hydrocarbon dispersion media are used in combination, for example, a commercially available mixed product such as trade name "Exxsol Heptane" (containing heptane and its isomeric hydrocarbons in 75 to 85% by mass content) available from Exxon Mobil Corporation, or the like can be used.

Since the polymerization temperature is easily controlled by removing heat of polymerization, it is preferred that the amount of the hydrocarbon dispersion medium used is set to 100 to 1,500 parts by mass, in particular to 200 to 1,400 parts by mass, relative to 100 parts by mass of the water-soluble ethylenically unsaturated monomer. In this connection, it is preferred that the amount of the hydrocarbon dispersion medium used in the case where the reversed-phase suspension polymerization is carried out by multi-stages is set to the above-mentioned proportion relative to 100 parts by mass of a water-soluble ethylenically unsaturated monomer to be firstly added to the hydrocarbon dispersion medium (namely, a water-soluble ethylenically unsaturated monomer to be used at the polymerization of the first stage).

A dispersion stabilizer usable in the production process of the present invention is one which contains a tall oil fatty acid ester. A tall oil fatty acid ester can be obtained by an esterification reaction of a tall oil fatty acid and an alcohol.

A tall oil fatty acid is generally obtained in a wood treatment such as pulp manufacturing by the Kraft process, and is a mixture containing oleic acid and linoleic acid, which are unsaturated fatty acids, as primary components along with other components such as a saturated fatty acid and rosin acid. As a tall oil fatty acid, a commercial product such as trade name "HARTALL FA-1", "HARTALL FA-1P" or "HARTALL FA-3S" available from Harima Chemicals, Inc. can be used. Two or more kinds of these tall oil fatty acids may be used in combination.

Examples of alcohol to be used for obtaining a tall oil fatty acid ester include sorbitol, sorbitan, sucrose, ethylene glycol, polyethylene glycol, glycerol, polyglycerol and the like. Two or more kinds of these alcohols may be used in combination.

A preferred tall oil fatty acid ester is one obtained by using sorbitol, sorbitan or polyethylene glycol as an alcohol for the esterification of a tall oil fatty acid, namely, a sorbitol tall oil fatty acid ester, a sorbitan tall oil fatty acid ester or a polyethylene glycol tall oil fatty acid ester.

Two or more kinds of tall oil fatty acid esters may be used in combination.

The dispersion stabilizer may be one which contains a polymeric dispersion stabilizer together with a tall oil fatty acid ester. Examples of the polymeric dispersion stabilizer include a maleic anhydride-modified polyethylene, a maleic anhydride-modified polypropylene, a maleic anhydride-modified ethylene/propylene copolymer, and the like. Two or more kinds of these polymeric dispersion stabilizers may be used in combination.

From the viewpoints of enabling the dispersion state of a water-soluble ethylenically unsaturated monomer in a hydrocarbon dispersion medium to be kept stable and enabling a dispersing effect commensurate with the amount of the dispersion stabilizer used to be attained, it is preferred that the amount of the dispersion stabilizer to be used is set to 0.1 to 30 parts by mass, in particular to 0.3 to 20 parts by mass, relative to 100 parts by mass of the water-soluble ethylenically unsaturated monomer. Also in the case where the dispersion stabilizer contains a polymeric dispersion stabilizer, with regard to the amount of the dispersion stabilizer used, from the viewpoint similar to the above, it is preferred that the total amount of the tall oil fatty acid ester and the polymeric dispersion stabilizer is set to 0.1 to 30 parts by mass, in particular to 0.3 to 20 parts by mass, relative to 100 parts by mass of the water-soluble ethylenically unsaturated monomer.

In the case of allowing a water-soluble ethylenically unsaturated monomer to undergo a reversed-phase suspension polymerization by multi-stages, it is preferred that the amount of the dispersion stabilizer to be used is set to the above-mentioned proportion relative to 100 parts by mass of a water-soluble ethylenically unsaturated monomer firstly added to the hydrocarbon dispersion medium (namely, a water-soluble ethylenically unsaturated monomer to be used at the time of the first stage polymerization).

It is usually preferred that the dispersion stabilizer is previously added to the hydrocarbon dispersion medium before added with a water-soluble ethylenically unsaturated monomer. In the case where a tall oil fatty acid ester and a polymeric dispersion stabilizer are used in combination as the dispersion stabilizer, a mixture thereof may be added to the hydrocarbon dispersion medium, and the two may be separately added to the hydrocarbon dispersion medium.

A radical polymerization initiator usable in the production process of the present invention is not particularly limited as long as the radical polymerization initiator is one usable in the reversed-phase suspension polymerization of a water-soluble ethylenically unsaturated monomer. Examples of the radical polymerization initiator include a persulfate such as potassium persulfate, ammonium persulfate and sodium persulfate; a peroxide such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypyvalate and hydrogen peroxide; an azo compound such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane } dihydrochloride, 2,2'-azobis{2-methyl-N-[1,1-bis(hydroxymethyl)-2-hydroxyeth yl]propionamide}, 2,2'-azobis[2-methyl-N-(2-hydroxyethyl)-propionamide] and 4,4'-azobis(4-cyanovaleric acid), and the like. Two or more kinds of radical polymerization initiators may be used in combination. In this connection, in the production process of the present invention, it is preferred that, among the exemplified radical polymerization initiators, potassium persulfate, ammonium persulfate, sodium persulfate and 2,2'-azobis(2-amidinopropane) dihydrochloride is used since these are easily available and are easy to be handled.

It is preferred that the amount of the radical polymerization initiator to be used is set to 0.005 to 1 mol, relative to 100 mol of the water-soluble ethylenically unsaturated monomer. In the case where the reversed-phase suspension polymerization is carried out by multi-stages, it is preferred that the amount of the radical polymerization initiator to be used is set so as to be within the above-mentioned range at the respective stages of the polymerization. In the case where the amount of the radical polymerization initiator used is less than 0.005 mol, there is a possibility that a very long period of time is required for the polymerization reaction. Conversely, in the case where the amount of the radical polymerization initiator used is greater than 1 mol, there is a possibility that a sudden polymerization reaction occurs and the control of the polymerization becomes difficult.

The radical polymerization initiator may be used, in combination with a reducing agent such as sodium sulfite, sodium hydrogen sulfite, ferrous sulfate or L-ascorbic acid, as a redox polymerization initiator.

It is usually preferred that the radical polymerization initiator is added to the water-soluble ethylenically unsaturated monomer before added to the hydrocarbon dispersion medium or an aqueous solution thereof.

In the reversed-phase suspension polymerization of a water-soluble ethylenically unsaturated monomer, the water-soluble ethylenically unsaturated monomer containing a radical polymerization initiator or an aqueous solution thereof is added to and dispersed in a hydrocarbon dispersion medium containing a dispersion stabilizer to be appropriately heated. At this time, to the water-soluble ethylenically unsaturated monomer containing a radical polymerization initiator or an aqueous solution thereof, a chain transfer agent for controlling the water-absorption ability of the target water-absorbent resin particles can be added. As the chain transfer agent, for example, a hypophosphite, a thiol, a thiolic acid, a secondary alcohol, an amine and the like can be used.

The reaction temperature of the reversed-phase suspension polymerization cannot be determined simply since it depends on the radical polymerization initiator to be used. It is usually preferred that the reaction temperature is set to 20 to 110°C, in particular to 40 to 90°C, because it is possible to enhance the productivity of water-absorbent resin particles by allowing the polymerization of a water-soluble ethylenically unsaturated monomer to proceed quickly and shortening the polymerization time and to make the polymerization reaction smooth by allowing the heat of polymerization to be more easily removed. The reaction time is typically set to 0.1 to 4 hours or so according to the reaction temperature.

When the polymerization of a water-soluble ethylenically unsaturated monomer is completed, a water-containing gelated product composed of water-absorbent resin particles dispersed in a hydrocarbon dispersion medium is formed in the reaction system. The target water-absorbent resin particles are usually obtained by performing a drying step in which water, the hydrocarbon dispersion medium and the like are removed from the reaction system. In the drying step, usually, a method of applying energy such as heat externally to the reaction system under normal pressure or under reduced pressure to remove the water content, the hydrocarbon dispersion medium and the like by distillation can be employed. At this time, for the purpose of enhancing the drying efficiency, it is also possible to introduce a stream of an inert gas such as nitrogen into the reaction system. In the case where the drying step is carried out under normal pressure, the drying temperature is preferably controlled to 60 to 250°C, more preferably controlled to 70 to 180°C, and particularly preferably controlled to 80 to 140°C. On the other hand, in the case where the drying step is carried out under reduced pressure, the drying temperature is preferably controlled to 60 to 100°C, and more preferably controlled to 70 to 90°C.

The production process of the present invention may further include the step of subjecting the water-absorbent resin particles obtained by the reversed-phase suspension polymerization to post-crosslinking. By applying a post-crosslinking step to the water-absorbent resin particles obtained, the water-absorption ability such as water-retention capacity of the water-absorbent resin particles is enhanced, and for example, the water-absorbent resin particles can be more suitably used in an absorbent article such as a disposable diaper.

The post-crosslinking step can be carried out by allowing a post-crosslinking agent to react with the water-absorbent resin particles formed.

Examples of the crosslinking agent usable in the post-crosslinking step (post-crosslinking agent) include a polyol such as ethylene glycol, propylene glycol, 1,4-butanediol, trimethylolpropane, glycerol, polyoxyethylene glycol, polyoxypropylene glycol and polyglycerol; a polyglycidyl compound such as ethylene glycol diglycidyl ether, polyethylene glycol diglycidyl ether, ethylene glycol triglycidyl ether, polyethylene glycol triglycidyl ether, glycerol diglycidyl ether, polyglycerol diglycidyl ether, glycerol triglycidyl ether, polyglycerol triglycidyl ether, propylene glycol polyglycidyl ether, polypropylene glycol polyglycidyl ether, glycerol polyglycidyl ether and polyglycerol polyglycidyl ether; a haloepoxy compound such as epichlorohydrin, epibromohydrin and $\alpha$-methylepichlorohydrin; a compound having two or more reactive functional groups such as an isocyanate compound including 2,4-tolylene diisocyanate, hexamethylene diisocyanate, and the like; an oxetane compound such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol and 3-butyl-3-oxetaneethanol; an oxazoline compound such as 1,2-ethylenebisoxazoline; a carbonate compound such as ethylene carbonate; a hydroxyalkylamide compound such as bis[N,N-di($\beta$-hydroxyethyl)]adipamide, and the like.

Among these post-crosslinking agents, it is preferred that a polyglycidyl compound such as ethylene glycol diglycidyl ether, polyethylene glycol diglycidyl ether, ethylene glycol triglycidyl ether, polyethylene glycol triglycidyl ether, glycerol diglycidyl ether, polyglycerol diglycidyl ether, glycerol triglycidyl ether, polyglycerol triglycidyl ether, propylene glycol polyglycidyl ether, polypropylene glycol polyglycidyl ether, glycerol polyglycidyl ether or polyglycerol polyglycidyl ether is used.

Two or more kinds of the post-crosslinking agents can also be used in combination.

From the viewpoint of effectively enhancing the water-absorption ability such as water-retention capacity of the water-absorbent resin particles, it is usually preferred that the amount of the post-crosslinking agent to be used is set to 0.001 to 1 mol, in particular to 0.005 to 0.5 mol, relative to 100 mol of the total amount of the water-soluble ethylenically unsaturated monomer used in the polymerization.

Although the application method of the post-crosslinking agent to the water-absorbent resin particles is not particularly limited, for example, the following methods can be employed.

(i) A method of directly adding a post-crosslinking agent to a water-containing gelated product composed of water-absorbent resin particles dispersed in a hydrocarbon dispersion medium after the completion of the reversed-phase suspension polymerization.

(ii) A method of adding a post-crosslinking agent solution to a water-containing gelated product composed of water-absorbent resin particles dispersed in a hydrocarbon dispersion medium after the completion of the reversed-phase suspension polymerization.

(iii) A method of misting and mixing powdered water-absorbent resin particles after subjected to a drying step with a post-crosslinking agent or a post-crosslinking agent solution atomized by spraying or the like under stirring.

The post-crosslinking agent solution usable in the above-mentioned application methods (ii) and (iii) is usually an aqueous solution or a hydrophilic organic solvent solution. Examples of the hydrophilic organic solvent for preparing the hydrophilic organic solvent solution include a lower alcohol such as methyl alcohol, ethyl alcohol, n-propyl alcohol, isopropyl alcohol and propylene glycol; a ketone such as acetone and methyl ethyl ketone; an ether such as diethyl ether, dioxane and tetrahydrofuran; an amide such as N,N-dimethylformamide; a sulfoxide such as dimethyl sulfoxide, and the like. Two or more kinds of these hydrophilic organic solvents may be used in combination. The hydrophilic organic solvent may be a mixed solvent prepared with water.

It is usually preferred that the post-crosslinking agent is added to water-absorbent resin particles in the presence of water. In this case, the amount of water is preferably controlled to 1 to 400 parts by mass, more preferably controlled to 5 to 200 parts by mass, and particularly preferably controlled to 10 to 100 parts by mass, relative to 100 parts by mass of the total amount of the water-soluble ethylenically unsaturated monomer used in the reversed-phase suspension polymerization.

The reaction temperature in the post-crosslinking step is preferably controlled to 50 to 250°C, more preferably controlled to 60 to 180°C, and particularly preferably controlled to 70 to 150°C. The reaction time required for the post-crosslinking step cannot be determined simply since it depends on the reaction temperature, the kind and amount of the post-crosslinking agent to be used and the like. It is usually preferred that the reaction time is set to 1 to 300 minutes, in particular to 5 to 200 minutes.

The water-absorbent resin particles obtained by the production process of the present invention exhibit satisfactory water-absorption ability and usually have a water-retention capacity of saline solution greater than or equal to 20 g/g. This water-retention capacity of saline solution can be controlled within a preferred range for heightening the absorption capacity of an absorbent material to be used for an absorbent article of 25 to 60 g/g or within a more preferred range for the same of 30 to 50 g/g, by adjusting the production condition of water-absorbent resin particles.

The water-absorbent resin particles obtained by the production process of the present invention have a preferred state of particles, in particular, a moderate particle size, and have a narrow particle size distribution. With regard to the moderate particle size, by adjusting the production condition, it is possible to allow the water-absorbent resin particles obtained by the production process of the present invention to have a median particle size suitable for an intended purpose. Specifically, in the case where the water-absorbent resin particles are used as absorbent products, for example, in the case of being used for a thin sheet-shaped article such as a water-blocking material for power cable, the median particle size of water-absorbent resin particles can be controlled to a relatively small particle size of 20 to 200 $\mu$m or so suitable for this purpose. In contrast, in the case where the water-absorbent resin particles are mixed with hydrophilic fiber to be used for the purpose of producing an absorbent material used for an absorbent article such as a disposable diaper, the median particle size of water-absorbent resin particles can be controlled to a relatively large particle size of 200 to 600 $\mu$m or so suitable for this purpose.

With regard to the narrow particle size distribution, it is possible to allow the water-absorbent resin particles obtained by the production process of the present invention to have a uniformity degree less than or equal to 3.0. This uniformity degree serves as an index for indicating the narrowness of particle size distribution, and it can be judged that the closer the degree is to 1.0 as the lower limit, the narrower the particle size distribution is. In this connection, by adjusting the condition of the process of the present invention, it is also possible to allow the uniformity degree to be controlled to a more preferred degree less than or equal to 2.6 and to a particularly preferred degree less than or equal to 2.4.

Since the water-absorbent resin particles obtained by the production process of the present invention have a narrow particle size distribution, problems which tend to occur in the case where water-absorbent resin particles have a wide particle size distribution hardly occur. For example, in the case where water-absorbent resin particles contain many small particles, problems such as the lowering in flowability and the dust generation occur, and also a significant loss of the water-absorbent resin particles tends to occur at the time of producing an absorbent material used for an absorbent article such as a disposable diaper because some of the particles readily pass through openings of a metal mesh. However, these problems are suppressed when the water-absorbent resin particles have a narrow particle size distribution. Moreover, in the case where water-absorbent resin particles contain many particles with a size of excessively large, the lowering in quality of an absorbent article or an absorbent product is easily caused. For example, in an absorbent article such as a disposable diaper, there is a possibility that large particles cause the lowering in water-absorption ability because an absorbent material containing large particles is liable to become hard when being compressed, and moreover, there is a possibility that large particles give a grainy unpleasant touch. However, these problems are suppressed when the water-absorbent resin particles have a narrow particle size distribution.

Respective numerical values concerning the water-retention capacity of saline solution, the median particle size and the uniformity degree of particle size distribution described above are values obtained by the measurement methods explained in the evaluation of examples mentioned later.

Since the water-absorbent resin particles obtained by the production process of the present invention achieve both satisfactory water-absorption ability and a preferred state of particles as described above, the water-absorbent resin particles are useful as materials for producing an absorbent material usable in various kinds of absorbent articles such as a disposable diaper, a sanitary napkin, an incontinence pad and a pet sheet, and various kinds of absorbent products such as a water-retaining material for soil, a water-blocking material for power cable and a dew-catcher.

The water-absorbent resin particles obtained by the production method of the present invention can be added with an additive such as a lubricant, a deodorant or an anti-bacterial agent according to the purpose of use and the like. Two or more kinds of additives may be used in combination.

EXAMPLES

Hereinafter, the present invention will be described in more detail on the basis of synthesis examples, examples and comparative examples, but the present invention is not limited only to these examples.

[Synthesis Example 1]

(Synthesis of sorbitan tall oil fatty acid ester)

A glass-made four-necked flask equipped with a stirring apparatus, a thermometer, a nitrogen gas inlet tube and an outlet tube was prepared. In this flask, 55 g of tall oil fatty acid (trade name "HARTALL FA-1" available from Harima Chemicals , Inc.), 37 g of an aqueous 70% by mass D-sorbitol solution, 0.4 g of sodium hydroxide and 0.3 g of phosphorous acid were charged. The contents of the flask were heated under normal pressure in a nitrogen gas environment and allowed to undergo a reaction for 6 hours at 220°C. After the completion of the reaction, the catalyst was removed by filtration, and furthermore, water was distilled off, whereupon 78 g of a sorbitan tall oil fatty acid ester was obtained.

[Example 1]

A 2-L, round-bottomed, 100 mm-inner diameter cylinder type separable flask equipped with a reflux condenser, a dropping funnel, a nitrogen gas inlet tube, a stirrer and a stirring blade was prepared. As the stirring blade, one having a blade diameter of 50 mm and having two rotating blade sections with four-piece-inclined paddles arranged in two stages was used. In this separable flask, 321 g (472 mL) of n-heptane was placed, and to this, 1.04 g of a sorbitan tall oil fatty acid ester obtained in the same manner as that in Synthesis Example 1 as a dispersion stabilizer and 0.92 g of a maleic anhydride-modified ethylene/propylene copolymer (trade name "Hi-WAX 1105A" available from Mitsui Chemicals, Inc.) as a polymeric dispersion stabilizer were added. The contents of the separable flask were heated to 80°C under stirring, and then were cooled to 65°C.

On the other hand, in a 500-mL Erlenmeyer flask, 92 g (1.03 mol) of an aqueous 80.5% by mass acrylic acid solution and 51.2 g of ion-exchanged water were placed, and to the contents, 102.9 g of an aqueous 30% by mass sodium hydroxide solution was added dropwise with external cooling to perform 75% by mole neutralization. To this, 0.27 g of hydroxyethyl cellulose (trade name "AW-15F" available from Sumitomo Seika Chemicals Co., Ltd.) as a thickener, 0.11 g (0.41 mmol) of potassium persulfate as a radical polymerization initiator, and 9.2 mg (0.05 mmol) of ethylene glycol diglycidyl ether as an internal-crosslinking agent were added, and these materials were dissolved to prepare an aqueous monomer solution.

The rotation number of the stirrer was set to 700 r/min, and to the above-mentioned separable flask, the total amount of the prepared aqueous monomer solution was added. Then, the interior of the separable flask was held at 45°C for 30 minutes while being replaced with nitrogen. Subsequently, the separable flask was immersed in a water bath at 70°C to elevate the temperature of the contents, and the polymerization was performed for 60 minutes.

After the polymerization, the rotation number of the stirrer was changed to 1,000 r/min, and the temperature of the contents of the separable flask was further elevated using an oil bath at 125°C. With this setup, water and n-heptane were subjected to an azeotropic distillation, and 125.7 g of water was removed out of the system while n-heptane was refluxed. Thereafter, the residue in the separable flask was added with 3.68 g of an aqueous 2% by mass solution of ethylene glycol diglycidyl ether as a post-crosslinking agent, and subjected to a post-crosslinking reaction for 2 hours at 80°C. Subsequently, water and n-heptane were removed by distillation from the inside of the separable flask, and the residue was dried, whereupon 95.8 g of spherical water-absorbent resin particles were obtained.

[Example 2]

A round-bottomed cylinder type separable flask similar to that prepared in Example 1 was prepared. In this separable flask, 321 g (472 mL) of n-heptane was placed, and to this, 1.04 g of a sorbitan tall oil fatty acid ester obtained in the same manner as that in Synthesis Example 1 as a dispersion stabilizer and 0.92 g of a maleic anhydride-modified ethylene/propylene copolymer (trade name "Hi-WAX 1105A" available from Mitsui Chemicals, Inc.) as a polymeric dispersion stabilizer were added. The contents of the separable flask were heated to 80°C under stirring, and then were cooled to 65°C.

On the other hand, two kinds of aqueous monomer solutions, namely, an aqueous monomer solution (in the present example, referred to as Aqueous monomer solution A) prepared in the same manner as that for the aqueous monomer solution used in Example 1 and an aqueous monomer solution (in the present example, referred to as Aqueous monomer solution B) different from the Aqueous monomer solution A were prepared.

The Aqueous monomer solution B was prepared in the following manner. First, in a 500-mL Erlenmeyer flask, 128.2 g (1.43 mol) of an aqueous 80.5% by mass acrylic acid solution and 30.5 g of ion-exchanged water were placed, and to the contents, 143.3 g of an aqueous 30% by mass sodium hydroxide solution was added dropwise with external cooling to perform 75% by mole neutralization. To this, 0.15 g (0.56 mmol) of potassium persulfate as a radical polymerization initiator and 12.8 mg (0.07 mmol) of ethylene glycol diglycidyl ether as an internal-crosslinking agent were added, and these materials were dissolved to obtain the Aqueous monomer solution B.

The rotation number of the stirrer was set to 700 r/min, and to the above-mentioned separable flask, the total amount of the Aqueous monomer solution A was added. Then, the interior of the separable flask was held at 45°C for 30 minutes while being replaced with nitrogen. Subsequently, the separable flask was immersed in a water bath at 70°C to elevate the temperature of the contents, and the first stage polymerization was performed for 60 minutes.

Next, the rotation number of the stirrer was changed to 1,000 r/min, and the total amount of the Aqueous monomer solution B was added to the slurry obtained by the first stage polymerization in the separable flask. Then, the interior of the separable flask was held at 22°C for 30 minutes while being replaced with nitrogen. Subsequently, the separable flask was immersed in a water bath at 70°C to elevate the temperature of the contents, and the second stage polymerization was performed for 30 minutes.

After the second stage polymerization, the temperature of the contents of the separable flask was further elevated using an oil bath at 125°C. With this setup, water and n-heptane were subjected to an azeotropic distillation, and 261.0 g of water was removed out of the system while n-heptane was refluxed. Thereafter, the residue in the separable flask was added with 3.96 g of an aqueous 2% by mass solution of ethylene glycol diglycidyl ether as a post-crosslinking agent, and subjected to a post-crosslinking reaction for 2 hours at 80°C. Subsequently, water and n-heptane were removed by distillation from the inside of the separable flask, and the residue was dried, whereupon 239.8 g of water-absorbent resin particles in the form of agglomerated spherical particles were obtained.

[Comparative Example 1]

A round-bottomed cylinder type separable flask similar to that prepared in Example 1 was prepared. In this separable flask, 321 g (472 mL) of n-heptane was placed, and to this, 0.92 g of a tetraglycerol stearic acid ester (trade name "RYOTO Polygly TS-4" available from Mitsubishi-Kagaku Foods Corporation) as a dispersion stabilizer and 0.92 g of a maleic anhydride-modified ethylene/propylene copolymer (trade name "Hi-WAX 1105A" available from Mitsui Chemicals, Inc.) as a polymeric dispersion stabilizer were added. The contents of the separable flask were heated to 80°C under stirring, and then were cooled to 55°C.

On the other hand, an aqueous monomer solution prepared in the same manner as that for the aqueous monomer solution used in Example 1 was prepared.

The rotation number of the stirrer was set to 700 r/min, and to the above-mentioned separable flask, the total amount of the prepared aqueous monomer solution was added. Then, the interior of the separable flask was held at 35°C for 30 minutes while being replaced with nitrogen. Subsequently, the separable flask was immersed in a water bath at 70°C to elevate the temperature of the contents, and the polymerization was performed for 60 minutes.

After the polymerization, the rotation number of the stirrer was changed to 1,000 r/min, and the temperature of the contents of the separable flask was further elevated using an oil bath at 125°C. With this setup, water and n-heptane were subjected to an azeotropic distillation, and 125.7 g of water was removed out of the system while n-heptane was refluxed. Thereafter, the residue in the separable flask was added with 3.68 g of an aqueous 2% by mass solution of ethylene glycol diglycidyl ether as a post-crosslinking agent, and subjected to a post-crosslinking reaction for 2 hours at 80°C. Subsequently, water and n-heptane were removed by distillation from the inside of the separable flask, and the residue was dried, whereupon 97.0 g of spherical water-absorbent resin particles were obtained.

[Comparative Example 2]

A round-bottomed cylinder type separable flask similar to that prepared in Example 1 was prepared. In this separable flask, 321 g (472 mL) of n-heptane was placed, and to this, 0.92 g of a tetraglycerol stearic acid ester (trade name "RYOTO Polygly TS-4" available from Mitsubishi-Kagaku Foods Corporation) as a dispersion stabilizer and 0.92 g of a maleic anhydride-modified ethylene/propylene copolymer (trade name "Hi-WAX 1105A" available from Mitsui Chemicals, Inc.) as a polymeric dispersion stabilizer were added. The contents of the separable flask were heated to 80°C under stirring, and then were cooled to 55°C.

On the other hand, two kinds of aqueous monomer solutions, namely, an aqueous monomer solution (in the present example, referred to as Aqueous monomer solution A) prepared in the same manner as that for the aqueous monomer solution used in Example 1 and an aqueous monomer solution (in the present example, referred to as Aqueous monomer solution B) prepared in the same manner as that for the Aqueous monomer solution B in Example 2 were prepared.

The rotation number of the stirrer was set to 450 r/min, and to the above-mentioned separable flask, the total amount of the Aqueous monomer solution A was added. Then, the interior of the separable flask was held at 35°C for 30 minutes while being replaced with nitrogen. Subsequently, the separable flask was immersed in a water bath at 70°C to elevate the temperature of the contents, and the first stage polymerization was performed for 60 minutes.

Next, the rotation number of the stirrer was changed to 1,000 r/min, and the total amount of the Aqueous monomer solution B was added to the slurry obtained by the first stage polymerization in the separable flask. Then, the interior of the separable flask was held at 25°C for 30 minutes while being replaced with nitrogen. Subsequently, the separable flask was immersed in a water bath at 70°C to elevate the temperature of the contents, and the second stage polymerization was performed for 30 minutes.

After the second stage polymerization, the temperature of the contents of the separable flask was further elevated using an oil bath at 125°C. With this setup, water and n-heptane were subjected to an azeotropic distillation, and 267.8 g of water was removed out of the system while n-heptane was refluxed. Thereafter, the residue in the separable flask was added with 3.96 g of an aqueous 2% by mass solution of ethylene glycol diglycidyl ether as a post-crosslinking agent, and subjected to a post-crosslinking reaction for 2 hours at 80°C. Subsequently, water and n-heptane were removed by distillation from the inside of the separable flask, and the residue was dried, whereupon 242.5 g of water-absorbent resin particles in the form of agglomerated spherical particles were obtained.

[Comparative Example 3]

A round-bottomed cylinder type separable flask similar to that prepared in Example 1 was prepared. In this separable flask, 378 g (472 mL) of cyclohexane was placed, and to this, 0.92 g of sorbitan monostearate (trade name "RHEODOL SP-10V" available from Kao Corporation) as a dispersion stabilizer and 0.92 g of a maleic anhydride-modified ethylene/propylene copolymer (trade name "Hi-WAX 1105A" available from Mitsui Chemicals, Inc.) as a polymeric dispersion stabilizer were added. The contents of the separable flask were heated to 80°C under stirring, and then were cooled to 55°C.

On the other hand, in a 500-mL Erlenmeyer flask, 92 g (1.03 mol) of an aqueous 80.5% by mass acrylic acid solution and 51.2 g of ion-exchanged water were placed, and to the contents, 102.9 g of an aqueous 30% by mass sodium hydroxide solution was added dropwise with external cooling to perform 75% by mole neutralization. To this, 0.11 g (0.41 mmol) of potassium persulfate as a polymerization initiator and 2.3 mg (0.01 mmol) of N,N'-methylene bisacrylamide as an internal-crosslinking agent were added, and these materials were dissolved to prepare an aqueous monomer solution.

The rotation number of the stirrer was set to 250 r/min, and to the above-mentioned separable flask, the total amount of the prepared aqueous monomer solution was added. Then, the interior of the separable flask was held at 35°C for 30 minutes while being replaced with nitrogen. Subsequently, the separable flask was immersed in a water bath at 70°C to elevate the temperature of the contents, and the polymerization was performed for 60 minutes.

After the polymerization, the rotation number of the stirrer was changed to 1,000 r/min, and the temperature of the contents of the separable flask was further elevated using an oil bath at 125°C. With this setup, water and cyclohexane were subjected to an azeotropic distillation, and 125.7 g of water was removed out of the system while cyclohexane was refluxed. Thereafter, the residue in the separable flask was added with 3.68 g of an aqueous 2% by mass solution of ethylene glycol diglycidyl ether as a post-crosslinking agent, and subjected to a post-crosslinking reaction for 2 hours at 80°C. Subsequently, water and cyclohexane were removed by distillation from the inside of the separable flask, and the residue was dried, whereupon 70.3 g of water-absorbent resin particles in the form of partially agglomerated spherical particles were obtained.

[Evaluation]

The water-absorbent resin particles obtained in examples and comparative examples were measured for the water-retention capacity of saline solution, the median particle size and the uniformity degree of particle size distribution. The measurement methods are as follows. The results are shown in Table 1.

(1) Water-retention capacity of saline solution

A 500-g portion of an aqueous 0.9% by mass sodium chloride solution (saline solution) was weighed into a 500-mL beaker, and 2.0 g of water-absorbent resin particles was added thereto while being stirred at 600 r/min and dispersed therein so as not to allow an unswollen lump to be generated. The resulting dispersion was kept stirred for 30 minutes so that the water-absorbent resin particles were sufficiently swollen. The swollen water-absorbent resin particles were poured into a cotton bag (cotton broad cloth No. 60, 100 mm in transversal length × 200 mm in longitudinal length) and the upper part of the cotton bag was tied with an elastic band. Then, the cotton bag was dehydrated for 1 minute using a dehydrator (product number "H-122" available from KOKUSAN ENSHINKI K.K.) in which the centrifugal force is set to 167 G, and the mass Wa (g) of the cotton bag containing the swollen gel after dehydration was measured. The same procedure was performed except that the water-absorbent resin particles are not added to the saline solution to measure the mass Wb (g) of an empty cotton bag in a wet state. The water-retention capacity of saline solution of water-absorbent resin particles was calculated from the following equation.

[Equation 1]

Water-retention capacity of saline solution (g/g) = [Wa - Wb] (g)/Mass of water-absorbent resin particles (g)

(2) Median particle size

The following two kinds of combinations of sieves were prepared.

(A) A combination of JIS standard sieves of a sieve having an opening of 500 μm, a sieve having an opening of 250 μm, a sieve having an opening of 180 μm, a sieve having an opening of 150 μm, a sieve having an opening of 106 μm, a sieve having an opening of 75 μm, a sieve having an opening of 45 μm and a receiving tray arranged in this order from the top.
(B) A combination of JIS standard sieves of a sieve having an opening of 850 μm, a sieve having an opening of 600 μm, a sieve having an opening of 500 μm, a sieve having an opening of 425 μm, a sieve having an opening of 300 μm, a sieve having an opening of 250 μm, a sieve having an opening of 150 μm and a receiving tray arranged in this order from the top.

The median particle size of water-absorbent resin particles was measured in the following procedure. In this case, a 50-g portion of water-absorbent resin particles to be measured are screened using a JIS standard sieve having a sieve opening of 250 μm. When the water-absorbent resin particles in an amount greater than or equal to 50% by mass pass through the sieve, the above-mentioned combination of sieves of (A) was used. On the other hand, when the water-absorbent resin particles in an amount greater than or equal to 50% by mass remain on the sieve, the above-mentioned combination of sieves of (B) was used.
In the combined uppermost sieve, about 50 g of water-absorbent resin particles were placed, and were subjected to classification by shaking the sieve for 10 minutes using a ro-tap type (Rotation and Tapping type) shaker. After the classification, each mass of water-absorbent resin particles remaining on the respective sieves was calculated in terms of mass percentage relative to the total amount of the water-absorbent resin particles, and the calculated values were integrated in order from the calculated value of larger particle size. The relations between the sieve openings and the integrated values of mass percentage of water-absorbent resin particles remaining on the sieve were plotted on a logarithmic-probability paper. The plots on the paper were connected with a straight line, and a particle size read at a point corresponding to the 50% by mass of the integrated mass percentage was defined as the median particle size.

(3) Uniformity degree of particle size distribution

In the measurement of the median particle size, the particle size (X1) corresponding to 15.9% by mass of the integrated mass percentage and the particle size (X2) corresponding to 84.1% by mass of the integrated mass percentage were determined to calculate the uniformity degree by the following equation. With regard to the uniformity degree, the closer the degree is to 1.0, the narrower the particle size distribution of water-absorbent resin particles is.

[Equation 2]

Uniformity degree = X1/X2

[Table 1]

Table 1

| | Median particle size [μm] | Uniformity degree of particle size distribution | Water-retention capacity of saline solution [g/g] |
|---|---|---|---|
| Example 1 | 102 | 2.1 | 43 |
| Example 2 | 440 | 1.8 | 36 |

(continued)

| | Median particle size [μm] | Uniformity degree of particle size distribution | Water-retention capacity of saline solution [g/g] |
|---|---|---|---|
| Comparative Example 1 | 57 | 3.3 | 35 |
| Comparative Example 2 | 390 | 3.8 | 37 |
| Comparative Example 3 | 220 | 3.5 | 33 |

According to Table 1, it has been found that the water-absorbent resin particles obtained in the examples have a moderate water-retention capacity (absorption capacity) and a particle size suitable for an absorbent article or for an absorbent product, and also is narrow in particle size distribution.

## Claims

1. A process for producing water-absorbent resin particles comprising the step of allowing a water-soluble ethylenically unsaturated monomer to undergo a reversed-phase suspension polymerization in the presence of a dispersion stabilizer, using a radical polymerization initiator in a hydrocarbon dispersion medium,
   wherein the dispersion stabilizer contains a tall oil fatty acid ester.

2. The process for producing water-absorbent resin particles according to claim 1, wherein the tall oil fatty acid ester is at least one kind of ester selected from the group consisting of a sorbitol tall oil fatty acid ester, a sorbitan tall oil fatty acid ester and a polyethylene glycol tall oil fatty acid ester.

3. The process for producing water-absorbent resin particles according to claim 1, wherein an amount of the dispersion stabilizer used relative to 100 parts by mass of the water-soluble ethylenically unsaturated monomer is set to 0.1 to 30 parts by mass.

4. The process for producing water-absorbent resin particles according to claim 1, wherein the reversed-phase suspension polymerization is performed by a multi-stage process of two or more stages.

5. The process for producing water-absorbent resin particles according to any one of claims 1 to 4, further comprising the step of subjecting the water-absorbent resin particles obtained by the reversed-phase suspension polymerization to post-crosslinking.

## Patentansprüche

1. Verfahren zur Erzeugung Wasser absorbierender Harzteilchen, umfassend den Schritt des Ermöglichens, dass ein wasserlösliches, ethylenisch ungesättigtes Monomer einer Umkehrphasen-Suspensionspolymerisation in Gegenwart eines Dispersionsstabilisators unterzogen wird, unter Verwendung eines Radikalpolymerisationsinitiators in einem Kohlenwasserstoffdispersionsmedium,
   wobei der Dispersionsstabilisator einen Tallölfettsäureester enthält.

2. Verfahren zur Erzeugung Wasser absorbierender Harzteilchen nach Anspruch 1, wobei der Tallölfettsäureester mindestens eine Art von Ester ist, der ausgewählt ist aus der Gruppe bestehend aus einem Sorbitol-Tallölfettsäureester, einem Sorbitan-Tallölfettsäureester und einem Polyethylenglykol-Tallölfettsäureester.

3. Verfahren zur Erzeugung Wasser absorbierender Harzteilchen nach Anspruch 1, wobei eine Menge des verwendeten Dispersionsstabilisators im Verhältnis zu 100 Massenteilen des wasserlöslichen, ethylenisch ungesättigten Monomers auf 0,1 bis 30 Massenteile festgelegt ist.

4. Verfahren zur Erzeugung Wasser absorbierender Harzteilchen nach Anspruch 1, wobei die Umkehrphasen-Suspensionspolymerisation durch einen mehrstufigen Prozess mit zwei oder mehr Stufen ausgeführt wird.

5. Verfahren zur Erzeugung Wasser absorbierender Harzteilchen nach einem der Ansprüche 1 bis 4, ferner umfassend den Schritt des Unterziehens der durch die Umkehrphasen-Suspensionspolymerisation erhaltenen Wasser absorbierenden Harzteilchen einer Nachvernetzung.

**Revendications**

1. Procédé de production de particules de résine absorbant l'eau comprenant l'étape consistant à laisser un monomère éthyléniquement insaturé soluble dans l'eau subir une polymérisation en suspension à phase inversée en présence d'un stabilisateur de dispersion, en utilisant un initiateur de polymérisation radicalaire dans un milieu de dispersion hydrocarboné,
le stabilisateur de dispersion contenant un ester d'acide gras de résine liquide.

2. Procédé de production de particules de résine absorbant l'eau selon la revendication 1, l'ester d'acide gras de résine liquide étant au moins un type d'ester choisi dans le groupe constitué par un ester d'acide gras de résine liquide de sorbitol, un ester d'acide gras de résine liquide de sorbitan et un ester d'acide gras de résine liquide de polyéthylène glycol.

3. Procédé de production de particules de résine absorbant l'eau selon la revendication 1, une quantité du stabilisateur de dispersion utilisé par rapport à 100 parties en masse du monomère éthyléniquement insaturé soluble dans l'eau étant fixée à 0,1 à 30 parties en masse.

4. Procédé de production de particules de résine absorbant l'eau selon la revendication 1, la polymérisation en suspension à phase inversée étant effectuée par un procédé à plusieurs étapes d'au moins deux étapes.

5. Procédé de production de particules de résine absorbant l'eau selon l'une quelconque des revendications 1 à 4, comprenant en outre l'étape consistant à soumettre les particules de résine absorbant l'eau obtenues par la polymérisation en suspension en phase inversée à une post-réticulation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 56026909 A
- JP 56131608 A
- JP 62172006 A